# EUROPÄISCHE PATENTSCHRIFT

(11) **EP 3 130 213 B1**
(45) Veröffentlichungstag und Bekanntmachung des Hinweises auf die Patenterteilung: **25.07.2018**
(21) Anmeldenummer: 16182935.3
(22) Anmeldetag: 05.08.2016
(51) Int. Cl.: A01D 41/127, G01N 21/85, G01N 33/10, G01N 21/359

(54) **MESSEINRICHTUNG ZUR UNTERSUCHUNG GEERNTETEN KORNS IN EINEM MÄHDRESCHER**
MEASURING DEVICE FOR EXAMINING HARVESTED GRAIN IN A COMBINE HARVESTER
DISPOSITIF DE MESURE DESTINÉ À ANALYSER DES GRAINS RÉCOLTÉS DANS UNE MOISSONNEUSE-BATTEUSE

(30) Priorität: 11.08.2015 DE 102015215299
(43) Veröffentlichungstag der Anmeldung: 15.02.2017
(73) Patentinhaber: Deere & Company, Moline, IL 61265 (US)
(72) Erfinder: Kormann, Georg, 66482 Zweibrücken (DE); Haiges, Wolfram, 71106 Magstadt (DE); Boydell, Broughton C., Pallamallawa, New South Wales 2399 (AU)
(74) Vertreter: Holst, Sönke

(56) Entgegenhaltungen:
- EP-A1- 1 894 461
- EP-A2- 1 639 879
- WO-A1-03/029792
- WO-A2-2007/034530

## Beschreibung

Die Erfindung betrifft eine Messeinrichtung zur Untersuchung geernteten Korns für einen Mähdrescher, umfassend:
eine Messkammer mit einem Einlass und einem Auslass für eine zu untersuchende Probe geernteten Korns, wobei die Messkammer so ausgebildet ist, dass die Probe im Betrieb entlang einer Durchlaufrichtung vom Einlass in die Messkammer und von dort in den Auslass gelangt
und ein Transmissionsspektrometer mit einem ersten Element in Form einer Lichtquelle und einem zweiten Element mit einem Aufnehmer für Licht, das von der Lichtquelle erzeugt und durch die Probe hindurch transmittiert wurde,
wobei der Aufnehmer mit einem Analysator zur wellenlängenaufgelösten Analyse des empfangenen Lichts verbunden ist und eine Halterung eines der Elemente des Transmissionsspektrometers gegenüber dem anderen Element durch einen Antrieb beweglich ist.

### Stand der Technik

Im Rahmen der Präzisionslandwirtschaft besteht ein Bedarf daran, Erntegut bereits während der Ernte auf bestimmte Eigenschaften hin zu untersuchen. Die Messwerte können z.B. zur Bereitstellung einer an die Ernteguteigenschaften angepassten Düngekarte elektronisch kartiert werden oder zur Festlegung des Marktpreises des Ernteguts oder zur qualitätsabhängigen Aufteilung des Ernteguts in unterschiedliche Behälter dienen. Im Falle eines Mähdreschers kann beispielsweise die Feuchtigkeit geernteten Korns oder dessen Proteingehalt erfasst werden. Als Messmethode steht insbesondere die Nahinfrarotspektroskopie zur Verfügung, bei der eine Probe des geernteten Korns durch die Schwerkraft oder einen zugehörigen Förderer in eine Messkammer eingebracht und dort mit breitbandigem Licht bestrahlt wird, dessen Spektrum sich (auch) in den nahen Infrarotbereich erstreckt. Das von der Probe reflektierte oder durch die Probe transmittierte Licht wird mittels eines Detektors aufgenommen und durch diesen wellenlängenabhängig untersucht. Hierzu sei beispielsweise auf die DE 10 2010 062 417 A1 und die dort erwähnten Referenzen verwiesen.

Da in der Regel schon Kalibriermesswerte aus Laboranalysen bereitstehen, wird die Transmissionsmessung in vielen Fällen gegenüber einer Reflexionsmessung bevorzugt. Allerdings ist die mittlere freie Weglänge des Lichts innerhalb der Kornprobe stark erntegutabhängig und liegt beispielsweise bei etwa 9 mm bei Raps und etwa 20 mm bei Mais oder Soja. Das bedeutet, dass es vorteilhaft ist, den Abstand zwischen der Lichtquelle und dem Detektor an die Art des Ernteguts anzupassen. Dazu wurde im Stand der Technik vorgeschlagen, zwei gegenüberliegende Wände der Messkammer relativ zueinander beweglich zu gestalten und jeweils eine der Wände mit der Lichtquelle und mit dem Detektor auszustatten (US 6 559 655 B1) oder die Lichtquelle an einer Wand zu befestigen und den Detektor an der gegenüber liegenden Wand verschiebbar zu lagern, sodass er von seiner Wand ausgehend mehr oder weniger weit in die Messkammer hineingeschoben werden kann, sei es von Hand oder mittels eines Antriebes (vgl. die als gattungsgemäß angesehene WO 2007/034530 A2).

### Problem

Ein Problem bei der Untersuchung von körnigem Erntegut innerhalb einer Messkammer liegt darin, dass sich innerhalb der Messkammer Brücken des Ernteguts bilden können oder sich das Erntegut dort sogar verstopfen oder verklemmen kann, auch wenn die Durchlassweite der Messkammer (wie in WO 2007/034530 A2 beschrieben) größer als die Entfernung zwischen Lichtquelle und Detektor sein sollte. Zur Vermeidung dieses Problems sind bei einem Feuchtesensor separate Elemente zur zwangsweisen Reinigung oder Entleerung der Messkammer vorgeschlagen worden (DE 197 44 485 A1), die jedoch recht (preis-) aufwändig sind. Die Verwendung des Transmissionsprinzips verbietet es zudem, einen die Messkammer mit dem Erntegut beaufschlagenden Förderer zur Vermeidung verstopfenden Ernteguts bis in die Messkammer hinein reichen zu lassen, denn dann würde er die Messung beeinträchtigen.

Die der Erfindung zu Grunde liegende Aufgabe wird darin gesehen, eine gegenüber dem Stand der Technik verbesserte Messeinrichtung bereitzustellen, die die erwähnten Nachteile nicht oder in einem verminderten Maß aufweist.

### Lösung

Die vorliegende Erfindung wird durch die Patentansprüche definiert.

Eine Messeinrichtung zur Untersuchung geernteten Korns für einen Mähdrescher umfasst eine Messkammer mit einem Einlass und einem Auslass für eine zu untersuchende Probe geernteten Korns, wobei die Messkammer so ausgebildet ist, dass die Probe im Betrieb entlang einer Durchlaufrichtung vom Einlass in die Messkammer und von dort in den Auslass gelangt. Ein Transmissionsspektrometer ist mit einem ersten Element in Form einer Lichtquelle und einem zweiten Element mit einem Aufnehmer für Licht, das von der Lichtquelle erzeugt und durch die Probe hindurch transmittiert wurde, ausgestattet. Der Aufnehmer ist mit einem Analysator zur wellenlängenaufgelösten Analyse des empfangenen Lichts verbunden und eine Halterung eines der Elemente des Transmissionsspektrometers gegenüber dem anderen Element ist durch einen Antrieb beweglich. Der Antrieb ist eingerichtet, die Halterung im Sinne einer Förderung der Probe in der Durchlaufrichtung und/oder entgegengesetzt dazu zu bewegen, um Brückenbildung und/oder Verstopfungen der Probe innerhalb der Messkammer aufzulösen oder zu vermeiden.

Mit anderen Worten wird die Halterung eines der Elemente des Transmissionsspektrometers durch den Antrieb nicht nur entlang der Laufrichtung des Lichts, d.h. quer zur Durchlaufrichtung der Probe durch die Messkammer, sondern (auch oder nur) entlang der Durchlaufrichtung bewegt, sei es in der Durchlaufrichtung oder ihr entgegen oder nacheinander in beiden erwähnten Richtungen. Der Antrieb dient somit nicht nur zur Verstellung der Halterung und somit des Elements, sondern auch oder nur dazu, die Probe aufzulockern und Brückenbildung und/oder Verstopfungen der Probe innerhalb der Messkammer aufzulösen oder zu vermeiden. Auf diese Weise wird die Messgenauigkeit verbessert und es erübrigt sich eine regelmäßige Kontrolle und Reinigung der Messkammer durch den Bediener.

Bei einer möglichen Ausführungsform ist der Antrieb eingerichtet, den Abstand zwischen den Elementen zur Anpassung an die (von der Art und insbesondere Färbung der Probe abhängige) mittlere Weglänge des Lichts durch die Probe zu variieren. Der Antrieb dient somit zum Verstellen des Elements in eine für die Messung geeignete Position. Bei einer anderen, weiter unten diskutierten Ausführungsform findet für diese Aufgabe ein weiterer Antrieb Verwendung, während der genannte Antrieb nur die Verstellung der Halterung in der Durchlaufrichtung übernimmt. Es sind auch gemischte Ausführungsformen denkbar, bei denen der Antrieb und der weitere Antrieb jeweils einen Teil der Verstellbewegung der Halterung in der Laufrichtung des Lichts bewirken.

Gemäß einer ersten Ausführungsform umfasst die Halterung eine in sich flexible Wand, an welcher das Element angebracht ist und die durch den Antrieb in eine peristaltische Bewegung bringbar ist. Die Wand ist demnach aus einem in sich flexiblen Material hergestellt und wird durch den Antrieb sukzessive in der Art einer fortschreitenden Welle mit einem oder mehreren Bergen und Tälern verformt. Das Element des Transmissionsspektrometers ist an der besagten, in sich flexiblen Wand angebracht und bewegt sich mit ihr.

Bei dieser Ausführungsform können an der von der Probe abgewandten Seite der in sich flexiblen Wand ein oder mehrere Nocken anliegen, die durch den Antrieb entlang einer Kurvenbahn beweglich sind, die sich zum Teil parallel zur Durchlaufrichtung erstreckt.

Der Aufnehmer und der Antrieb können mit einer Steuereinrichtung verbunden sein, welche betreibbar ist, den Zeitpunkt der Aufnahme eines Spektrums zwecks Anpassung an die mittlere Weglänge des Lichts durch die Probe abhängig von der Position des Nocken oder der Nocken zu kontrollieren. Es findet somit die Aufnahme eines Spektrums statt, wenn sich die Wand mit der Halterung und dem einen Element des Transmissionsspektrometers in einem geeigneten Abstand vom anderen Element des Transmissionsspektrometers befindet. Sollte die Amplitude der Bewegung der Wand nicht zur Anpassung an die benötigten Weglängen des Lichts durch die Probe ausreichen, kann alternativ oder zusätzlich die Position der Kurvenbahn zur Anpassung an die mittlere Weglänge des Lichts durch die Probe durch den bereits erwähnten, weiteren Antrieb verstellbar sein.

Bei einer zweiten Ausführungsform ist die Halterung durch den Antrieb entlang einer Kurvenbahn beweglich. Die Kurvenbahn kann eine beliebige Form haben, z.B. kreisförmig, elliptisch, rechteckig, dreieckig oder geradlinig (d.h. in Durchlaufrichtung oder in einem Winkel dazu orientiert).

Auch bei dieser Ausführungsform können der Aufnehmer und der Antrieb mit einer Steuereinrichtung verbunden sein, welche betreibbar ist, den Zeitpunkt der Aufnahme eines Spektrums zwecks Anpassung an die mittlere Weglänge des Lichts durch die Probe abhängig von der Position der Halterung entlang der Kurvenbahn zu kontrollieren. Es findet somit die Aufnahme eines Spektrums statt, wenn sich die Halterung mit dem einen Element des Transmissionsspektrometers in einem geeigneten Abstand vom anderen Element des Transmissionsspektrometers befindet. Sollte die Amplitude der Bewegung der Halterung in der Laufrichtung des Lichts zur nicht zur Anpassung an die benötigten Weglängen des Lichts durch die Probe ausreichen, kann alternativ oder zusätzlich die Position der Kurvenbahn zur Anpassung an die mittlere Weglänge des Lichts durch die Probe durch den bereits erwähnten, weiteren Antrieb verstellbar sein.

Die Halterung kann gekröpft sein und einen sich entlang der Laufrichtung des Lichts erstreckenden, ersten Abschnitt, in dem das Element angeordnet ist, und einen quer dazu und quer zur Durchlaufrichtung verlaufenden, zweiten Abschnitt umfassen. Dieser Abschnitt kann sich durch eine Seitenwand der Messkammer erstrecken.

### Ausführungsbeispiele

In den Zeichnungen sind zwei nachfolgend näher beschriebene Ausführungsbeispiele der Erfindung dargestellt. Es zeigen:
- Fig. 1: eine schematische seitliche Ansicht eines Mähdreschers mit einer Messeinrichtung,
- Fig. 2: eine schematische seitliche Ansicht einer ersten Ausführungsform einer Messeinrichtung,
- Fig. 3: eine schematische seitliche Ansicht einer zweiten Ausführungsform einer Messeinrichtung, und
- Fig. 4: eine Draufsicht auf die Messeinrichtung der Figur 3.

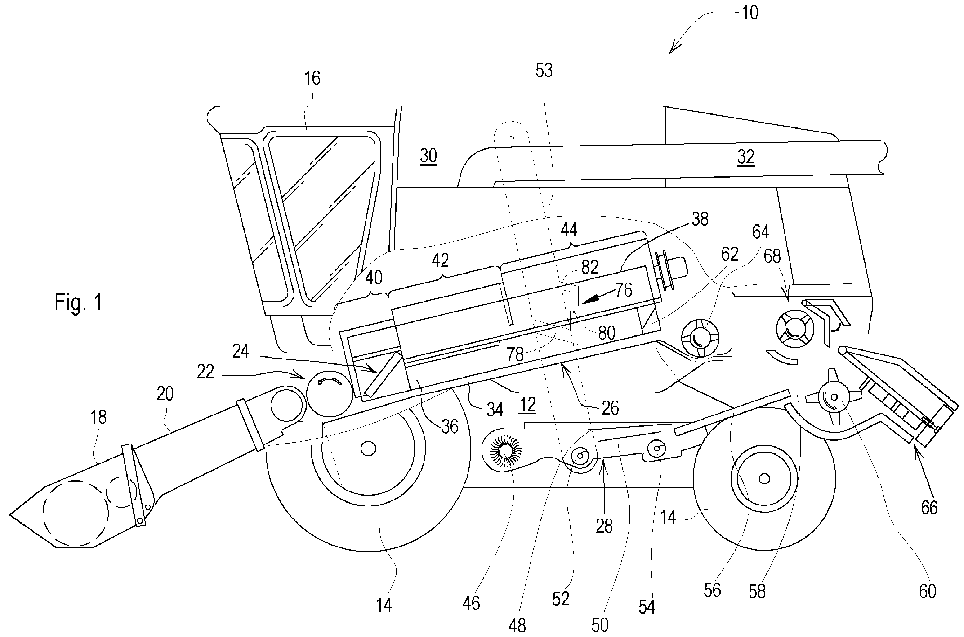

Die Figur 1 zeigt einen Mähdrescher 10 mit einem tragenden Rahmen 12, der sich auf angetriebenen, vorderen Rädern 14 und lenkbaren, hinteren Rädern 14 abstützt. Der Betrieb des Mähdreschers 10 wird von einer Bedienerkabine 16 aus kontrolliert. Ein Schneidwerk 18 wird verwendet, um Korn enthaltendes Erntegut zu ernten und es einem Schrägförderer 20 zuzuführen. Das geerntete Gut wird durch den Schrägförderer 20 einer Leittrommel 22 zugeführt. Die Leittrommel 22 leitet das Erntegut durch einen Einlassübergangsabschnitt 24 zu einer axialen Erntegutbearbeitungseinrichtung 26. Im Folgenden beziehen sich Richtungsangaben, wie vorn und hinten, auf die Vorwärtsrichtung des Mähdreschers 10.

Die Erntegutbearbeitungseinrichtung 26 umfasst ein Rotorgehäuse 34 und einen darin angeordneten Rotor 36. Der Rotor 36 umfasst eine hohle Trommel 38, an der Gutbearbeitungselemente für einen Beschickungsabschnitt 40, einen Dreschabschnitt 42 und einen Trennabschnitt 44 befestigt sind. Der Beschickungsabschnitt 40 ist an der Vorderseite der axialen Erntegutbearbeitungseinheit 26 angeordnet. In Längsrichtung stromab und rückwärtig des Beschickungsabschnitts 40 liegen der Dreschabschnitt 42 und der Trennabschnitt 44. Die Trommel 38 ist im Beschickungsabschnitt 40 kegelstumpfförmig. Der Dreschabschnitt 42 umfasst einen kegelstumpfförmigen vorderen Abschnitt und einen zylindrischen rückwärtigen Abschnitt. Am Ende der axialen Erntegutbearbeitungseinheit 26 befindet sich der zylindrische Trennabschnitt 44 der Trommel 38. Anstelle einer axialen Erntegutbearbeitungseinheit 26 kann auch eine tangentiale Dreschtrommel und eine ihr folgende axiale Trenneinrichtung oder Strohschüttler verwendet werden.

Korn und Spreu, die durch einen dem Dreschabschnitt 42 zugeordneten Dreschkorb und ein dem Trennabschnitt 44 zugeordneten Trennrost fallen, werden einem Reinigungssystem 28 mit einem Gebläse 46 und in eine Schwingbewegung versetzbaren Lamellensieben 48, 50 zugeführt. Das Reinigungssystem 28 entfernt die Spreu und führt das saubere Korn über einen Schneckenförderer 52 einem Körnerelevator 53 zu. Der Körnerelevator 53 legt das saubere Korn in einem Korntank 30 ab. Das saubere Korn im Korntank 30 kann durch einen Entladeschneckenförderer 32 auf einen Kornwagen, Anhänger oder Lastwagen entladen werden. Am rückwärtigen Ende des unteren Lamellensiebs 50 verbleibendes Erntegut wird mittels eines Schneckenförderers 54 und eines Überkehrförderers (nicht gezeigt) wieder der Erntegutbearbeitungseinrichtung 26 oder einem separaten Nachdrescher (nicht gezeigt) zugeführt. Die am rückwärtigen Ende des oberen Lamellensiebs 48 abgegebenen Erntegutreste, die im Wesentlichen aus Kaff (Spreu) und kleinen Strohteilchen bestehen, werden durch einen Schwingbodenförderer 56 nach hinten in einen Einlass 58 eines Strohhäckslers 60 gefördert.

Gedroschenes, den Trennabschnitt 44 verlassendes Stroh wird durch einen Auslass 62 aus der Erntegutbearbeitungseinrichtung 26 ausgestoßen und einer Auswurftrommel 64 zugeführt. Die Auswurftrommel 64 wirft das Stroh nach hinten aus. Rückwärtig der Auswurftrommel 64 und etwa in vertikaler Höhe ihrer Drehachse befindet sich ein oberschlächtig arbeitender Trommelförderer 68, der das Stroh entweder nach hinten auswirft (Schwadablage) oder dem Strohhäcksler 60 zuführt, der die Häcksel einem aktiven Verteiler 66 zuführt.

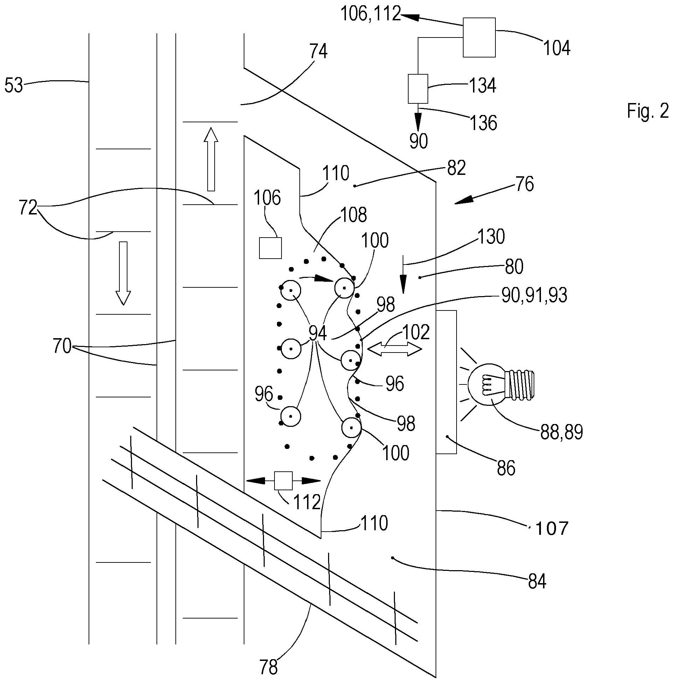

Wie in der Figur 2 gezeigt, ist der Körnerelevator 53 als Paddelförderer ausgeführt. An einem flexiblen, um einen unteren und einen oberen Umlenkpunkt umlaufenden, angetriebenen Zugmittel 70 in Form einer Kette o.ä. sind in regelmäßigen Abständen Paddel 72 angebracht. Das hochlaufende Trum des Zugmittels fördert das saubere Korn nach oben. Die hoch- und herunterlaufenden Trums können durch eine Trennwand getrennt werden. In der Seitenwand des Körnerelevators 53 ist eine Öffnung 74 vorgesehen, durch die eine Probe des Korns in eine Messeinrichtung 76 gelangen kann, in der die Probe durch ein Transmissionsspektrometer auf seine Inhaltsstoffe, wie Wassergehalt, Protein etc. untersucht werden kann. Die Probe wird anschließend durch einen Förderer 78 in das hoch- oder herunterlaufende Trum des Körnerelevators 53 eingebracht. Die Messeinrichtung 76 könnte auch an einer beliebigen anderen Stelle des Mähdreschers 10 angebracht werden, an der sauberes Korn entnommen werden kann, wie am Schneckenförderer 52 oder am Auslass des Körnerelevators 53 oder an beliebiger Stelle einer Korntankbefüllschnecke (nicht gezeigt).

Die Messeinrichtung 76 umfasst eine Messkammer 80 mit einem oberen Einlass 82, durch den kontinuierlich (oder sukzessive, z.B. unter Verwendung einer oberen Einlassschleuse, nicht gezeigt) die Probe in die Messkammer 80 gelangt. Die Probe durchläuft im Betrieb die Messkammer 80 in einer Durchlaufrichtung 130 nach unten und gelangt in einen unteren Auslass 84, von dem aus sie durch den Förderer 78 wieder abgefördert wird. In der Messkammer 80 ist ein im nahen Infrarotbereich arbeitendes Transmissionsspektrometer angebracht, das ein erstes Element 88 in Form einer Lichtquelle 89 (z.B. Halogenlampe oder Leuchtdiodenanordnung) aufweist, die durch eine Fensterscheibe 86 den Innenraum der Messkammer 80 ausleuchtet. Das Transmissionsspektrometer umfasst weiterhin ein zweites Element 90 in Form eines Aufnehmers 91 für das Licht, das durch die in der Messkammer 80 enthaltene Probe transmittiert (hindurchgelassen) wurde. Der Aufnehmer 91 könnte eine Fensterscheibe und/oder Sammellinse umfassen und leitet das Licht zu einem Analysator 134, der das Licht wellenabhängig zerlegt und die den Wellenlängen zugehörigen Intensitäten bestimmt, wozu auf den Stand der Technik nach US 5 751 421 A, DE 199 22 867 A1, WO 2007/034530 A2, DE 10 2010 062 417 A1 und DE 10 2011 054 841 A1 verwiesen sei. Eine elektronische Steuereinrichtung 104 ermittelt in an sich bekannter Weise anhand von Kalibrierdaten und den gemessenen, wellenlängenabhängigen Intensitäten den Gehalt der Probe an den erwähnten Inhaltsstoffen. Der Analysator 134 kann räumlich dem Aufnehmer 91 direkt benachbart oder darin integriert oder im Abstand davon angeordnet und durch einen Lichtleiter 136 damit verbunden sein.

Während die Wand 107, an der das erste Element 88 und die Fensterscheibe 86 befestigt sind, in sich starr ist, besteht die gegenüberliegende Wand 108 der Messkammer 80 aus einem in sich flexiblen Material, wie Gummi oder Kunststoff. An dieser Wand 108 aus in sich flexiblem Material ist das zweite Element 90 befestigt und bewegt sich mit der Wand 108 mit, wenn diese durch Nocken 94, die durch einen Antrieb 106 kontinuierlich entlang einer Kurvenbahn 96 bewegt werden, in eine peristaltische Bewegung versetzt wird, bei der jeweils Teile der Wand 108 durch die Nocken 94 sukzessive nach außen gedrückt werden und dort Berge 100 bilden, während die Wand 108 dazwischen durch den Druck der Probe Täler 98 bildet. Die Kurvenbahn 96 ist in der dargestellten Ausführungsform etwa elliptisch, könnte auch die Form einer Stadionlaufbahn haben mit geraden, vertikalen Abschnitten, die durch Halbkreise verbunden sind, oder eine beliebige andere Form haben. Die Wand 108 geht ober- und unterhalb der Messkammer 80 jeweils in starre Wände 110 über bzw. ist damit verbunden.

Die durch die Wand 108 gebildete Halterung 93 des zweiten Elements 90 bewegt sich somit durch den Antrieb 106 kontinuierlich in Richtung des Pfeils 102 in Richtung auf das erste Element 88 hin und wieder davon hinweg. Zudem wird die besagte Halterung 93 im Sinne einer Förderung der Probe in der Durchlaufrichtung 130 durch die Messkammer 80 bewegt, was eine Brückenbildung und/oder Verstopfungen der Probe innerhalb der Messkammer 80 auflöst oder vermeidet. Die Bewegung der Wand 108 kann dazu dienen, durch die Steuereinrichtung 104, der die Position des Antriebs 106 und somit der Nocken 94 durch einen entsprechenden Sensor und/oder von der Ansteuerung des z.B. als Schritt- oder Servomotors ausgeführten Antriebs 106 her bekannt ist, eine Messung durch das Transmissionsspektrometer dann auszulösen, wenn die Elemente 88, 90 in einem geeigneten, der Weglänge des Lichts durch die Probe angepassten Abstand voneinander sind. Sollte die Amplitude der Bewegung des zweiten Elements 90 hierfür nicht ausreichen, kann die Steuereinrichtung 104 einen weiteren Antrieb 112 veranlassen, bei Bedarf die ganze Kurvenbahn 96 (und mit ihr die Nocken 94 und somit auch die Wand 108) durch einen weiteren Antrieb 112 in Richtung des Pfeils 102 zu verstellen. Selbstverständlich könnte das erste Element 88 an der Wand 108 angebracht werden, während das zweite Element 90 dann an der festen Wand 107 befestigt wird.

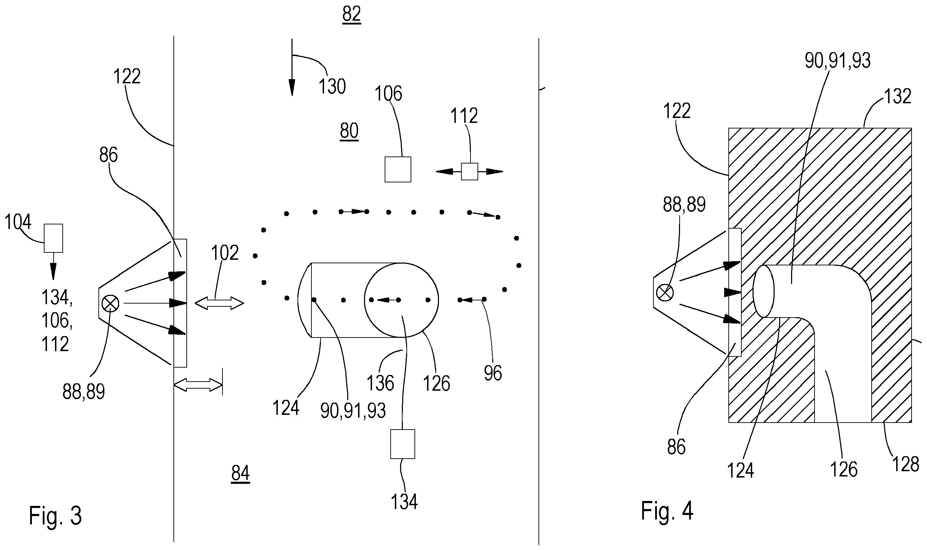

Bei der in den Figuren 3 und 4 gezeigten, zweiten Ausführungsform, bei der mit der ersten Ausführungsform übereinstimmende Elemente mit denselben Bezugszeichen versehen sind, bewegt sich das gesamte zweite Element 90 mit seiner Halterung 93 auf einer Kurvenbahn 96, die ebenfalls näherungsweise elliptisch ist und sich zum Teil entlang des die Laufrichtung des Lichts vom ersten Element 88 zum zweiten Element 90 darstellenden Pfeils 102 erstreckt und zum Teil quer dazu erstreckt, d.h. entgegen der Durchlaufrichtung 130 der Probe des Ernteguts durch die Messkammer 80, welche in der Figur 3 von oben nach unten und in der Figur 4 senkrecht zur Zeichenebene verläuft. Das zweite Element 90 des Transmissionsspektrometers bewegt sich somit nicht nur in der Laufrichtung des Lichts (Pfeil 102), sondern auch (im linken Umkehrpunkt der Figur 3) entgegen der Durchlaufrichtung 130 und (im rechten Umkehrpunkt der Figur 3) in der Durchlaufrichtung 130, was die Probe auflockert und Verstopfungen und Brückenbildung vermeidet. Die Drehrichtung der Halterung 93 kann in der Figur 3 auch umgedreht werden.

Wie bei der ersten Ausführungsform kann die Steuereinrichtung 104, der die Position des Antriebs 106 und somit der Nocken 94 durch einen entsprechenden Sensor und/oder von der Ansteuerung des z.B. als Schritt- oder Servomotors ausgeführten Antriebs 106 her bekannt ist, eine Messung durch das Transmissionsspektrometer genau dann auslösen, wenn die Elemente 88, 90 in einem geeigneten, der Weglänge des Lichts durch die Probe angepassten Abstand voneinander sind. Sollte die Amplitude der Bewegung des zweiten Elements 90 entlang der Kurvenbahn 96 (d.h. entlang des Pfeils 102) hierfür nicht ausreichen, kann die Steuereinrichtung 104 einen weiteren Antrieb 112 veranlassen, bei Bedarf die ganze Kurvenbahn 96 (und mit ihr das zweite Element 90) durch einen weiteren Antrieb 112 in Richtung des Pfeils 102 zu verstellen. Selbstverständlich könnte das erste Element 88 entlang der Kurvenbahn 96 bewegt werden, während das zweite Element 90 dann starr an der festen Wand 122 befestigt wird.

Das zweite Element 90 ist in sich gekröpft und weist einen ersten Abschnitt 124 auf, der sich entlang des Pfeils 102 erstreckt, und einen zweiten Abschnitt 126, der sich senkrecht dazu und zur Durchflussrichtung 130 erstreckt. Der zweite Abschnitt 126 erstreckt sich durch eine seitliche Wand 128 der Messkammer 80, die einer vierten Wand 132 gegenüber liegt.

## Patentansprüche

1. Messeinrichtung (80) zur Untersuchung geernteten Korns für einen Mähdrescher (10), umfassend:
eine Messkammer (80) mit einem Einlass (82) und einem Auslass (84) für eine zu untersuchende Probe geernteten Korns, wobei die Messkammer (80) so ausgebildet ist, dass die Probe im Betrieb entlang einer Durchlaufrichtung (130) vom Einlass (82) in die Messkammer (80) und von dort in den Auslass (84) gelangt
und ein Transmissionsspektrometer mit einem ersten Element (88) in Form einer Lichtquelle (89) und einem zweiten Element (90) mit einem Aufnehmer (91) für Licht, das von der Lichtquelle (89) erzeugt und durch die Probe hindurch transmittiert wurde,
wobei der Aufnehmer (91) mit einem Analysator (134) zur wellenlängenaufgelösten Analyse des empfangenen Lichts verbunden ist und eine Halterung (93) eines der Elemente (88, 90) des Transmissionsspektrometers gegenüber dem anderen Element (90, 88) durch einen Antrieb (106) beweglich ist,
**dadurch gekennzeichnet, dass** der Antrieb (106) eingerichtet ist, die Halterung (93) im Sinne einer Förderung der Probe in der Durchlaufrichtung (130) und/oder entgegengesetzt dazu zu bewegen, um Brückenbildung und/oder Verstopfungen der Probe innerhalb der Messkammer (80) aufzulösen oder zu vermeiden.

2. Messeinrichtung (80) nach Anspruch 1, wobei der Antrieb (106) eingerichtet ist, den Abstand zwischen den Elementen (88, 90) zur Anpassung an die mittlere Weglänge des Lichts durch die Probe zu variieren.

3. Messeinrichtung (80) nach Anspruch 1 oder 2, wobei die Halterung (93) eine in sich flexible Wand (108) umfasst, an welcher das Element (88, 90) angebracht ist und die durch den Antrieb (16) in eine peristaltische Bewegung bringbar ist.

4. Messeinrichtung (80) nach Anspruch 3, wobei an der von der Probe abgewandten Seite der in sich flexiblen Wand (108) ein oder mehrere Nocken (94) anliegen, die durch den Antrieb (106) entlang einer Kurvenbahn (96) beweglich sind, die sich zum Teil parallel zur Durchlaufrichtung (130) erstreckt.

5. Messeinrichtung (80) nach Anspruch 4, wobei der Analysator (134) und der Antrieb (106) mit einer Steuereinrichtung (104) verbunden sind und die Steuereinrichtung (104) betreibbar ist, den Zeitpunkt der Aufnahme eines Spektrums zwecks Anpassung an die mittlere Weglänge des Lichts durch die Probe abhängig von der Position des oder der Nocken (94) zu kontrollieren und/oder wobei die Position der Kurvenbahn (96) zwecks Anpassung an die mittlere Weglänge des Lichts durch die Probe durch einen weiteren Antrieb (112) verstellbar ist.

6. Messeinrichtung (80) nach einem der Ansprüche 1 oder 2, wobei die Halterung (93) durch den Antrieb (106) entlang einer Kurvenbahn (96) beweglich ist.

7. Messeinrichtung (80) nach Anspruch 6, wobei der Analysator (134) und der Antrieb (106) mit einer Steuereinrichtung (104) verbunden sind und die Steuereinrichtung (104) betreibbar ist, den Zeitpunkt der Aufnahme eines Spektrums zur Anpassung an die mittlere Weglänge des Lichts durch die Probe abhängig von der Position der Halterung (93) entlang der Kurvenbahn (96) zu kontrollieren und/oder wobei die Position der Kurvenbahn (96) durch einen weiteren Antrieb (112) zur Anpassung an die mittlere Weglänge des Lichts durch die Probe verstellbar ist.

8. Messeinrichtung (80) nach Anspruch 7, wobei die Halterung (93) gekröpft ist und einen ersten Abschnitt (124), in dem das Element angeordnet ist, und einen quer dazu und quer zur Durchlaufrichtung verlaufenden, zweiten Abschnitt (126) umfasst.

9. Mähdrescher (10) mit einer Messeinrichtung (80) nach einem der vorhergehenden Ansprüche.

## Claims

1. Measuring device (80) for examining harvested grain for a combine harvester (10), comprising:
a measuring chamber (18) having an inlet (82) and an outlet (84) for a sample of harvested grain to be examined, the measuring chamber (80) being formed such that during operation the sample passes along a passage direction (130) from the inlet (82) into the measuring chamber (80) and from there into the outlet (84)
and a transmission spectrometer having a first element (88) in the form of a light source (89) and a second element (90) having a detector (91) for light which has been generated by the light source (89) and has been transmitted through the sample,
the detector (91) being connected to an analyser (134) for the wavelength-resolved analysis of the received light, and a holder (93) of one of the elements (88, 90) of the transmission spectrometer being movable with respect to the other element (90, 88) by a drive (106),
**characterized in that** the drive (106) is configured to move the holder (93) with the effect of conveying the sample in and/or opposite to the passage direction (130), in order to break up or to avoid bridging and/or blockages of the sample within the measuring chamber (80).

2. Measuring device (80) according to Claim 1, wherein the drive (106) is configured to vary the distance between the elements (88, 90) in order to adapt to the average wavelength of the light through the sample.

3. Measuring device (80) according to Claim 1 or 2, wherein the holder (93) comprises an intrinsically flexible wall (108), to which the element (88, 90) is attached and which can be caused to make a peristaltic movement by the drive (16).

4. Measuring device (80) according to Claim 3, wherein one or more cams (94), which are movable by the drive (106) along a curved path (96) which to some extent extends parallel to the passage direction (130), bear on the side of the intrinsically flexible wall (108) which faces away from the sample.

5. Measuring device (80) according to Claim 4, wherein the analyser (134) and the drive (106) are connected to a control device (104), and the control device (104) can be operated to control the time at which a spectrum is detected, for the purpose of adaptation to the average wavelength of the light through the sample, depending on the position of the cam(s) (94), and/or wherein the position of the curved path (96) can be adjusted by a further drive (112) for the purpose of adaptation to the average wavelength of the light through the sample.

6. Measuring device (80) according to either of Claims 1 and 2, wherein the holder (93) is movable along a curved path (96) by the drive (106).

7. Measuring device (80) according to Claim 6, wherein the analyser (134) and the drive (106) are connected to a control device (104), and the control device (104) can be operated to control the time at which a spectrum is detected for adaptation to the average wavelength of the light through the sample, depending on the position of the holder (93) along the curved path (96), and/or wherein the position of the curved path (96) can be adjusted by a further drive (112) for adaptation to the average wavelength of the light through the sample.

8. Measuring device (80) according to Claim 7, wherein the holder (93) is bent at an angle and comprises a first section (124) in which the element is arranged and a second section (106) extending transversely thereto and transversely to the passage direction.

9. Combine harvester (10) having a measuring device (80) according to one of the preceding claims.

## Revendications

1. Dispositif de mesure (80) destiné à analyser le grain récolté dans une moissonneuse-batteuse (10), comprenant :
une chambre de mesure (80) ayant une entrée (82) et une sortie (84) destinées à un échantillon de grain récolté à analyser, dans lequel la chambre de mesure (80) est configurée de manière à ce que, pendant le fonctionnement, l'échantillon passe de l'entrée (82) vers la chambre de mesure (80) et de là vers la sortie (84) suivant une direction d'écoulement (130), et un spectromètre de transmission comprenant un premier élément (88) sous la forme d'une source de lumière (89) et un second élément (90) comportant un capteur (91) destiné à la lumière générée par la source de lumière (89) et transmise à travers l'échantillon,
dans lequel le capteur (91) est relié à un analyseur (134) destiné à effectuer une analyse résolue en longueur d'onde de la lumière reçue et un support (93) de l'un des éléments (88, 90) du spectromètre de transmission peut être déplacé par rapport à l'autre élément (90, 88) par un dispositif d'entraînement (106),
**caractérisé en ce que** le dispositif d'entraînement (106) est conçu pour déplacer le support (93) dans la direction d'écoulement (130) et/ou en sens inverse pour transporter l'échantillon afin d'éliminer ou d'éviter le pontage et/ou le colmatage de l'échantillon à l'intérieur de la chambre de mesure (80).

2. Dispositif de mesure (80) selon la revendication 1, dans lequel le dispositif d'entraînement (106) est conçu pour faire varier la distance entre les éléments (88, 90) pour l'adaptation à la longueur moyenne du trajet de la lumière à travers l'échantillon.

3. Dispositif de mesure (80) selon la revendication 1 ou 2, dans lequel le support (93) comprend une paroi (108) intrinsèquement flexible à laquelle est fixé l'élément (88, 90) et qui peut être mise en mouvement péristaltique par le dispositif d'entraînement (16).

4. Dispositif de mesure (80) selon la revendication 3, dans lequel une ou plusieurs cames (94), qui peuvent être déplacées par le dispositif d'entraînement (106) suivant un trajet courbe (96) s'étendant en partie parallèlement à la direction de passage (130), reposent sur le côté de la paroi intrinsèquement flexible (108) qui est tourné à l'opposé de l'échantillon.

5. Dispositif de mesure (80) selon la revendication 4, dans lequel l'analyseur (134) et le dispositif d'entraînement (106) sont reliés à un dispositif de commande (104) et le dispositif de commande (104) peut être mis en fonctionnement pour contrôler l'instant d'acquisition d'un spectre à des fins d'adaptation à la longueur moyenne du trajet de la lumière à travers l'échantillon en fonction de la position de la ou des cames (94) et/ou dans lequel la position du trajet courbe (96) peut être réglée par un autre dispositif d'entraînement (112) à des fins d'adaptation à la longueur moyenne du trajet de la lumière à travers l'échantillon.

6. Dispositif de mesure (80) selon l'une des revendications 1 ou 2, dans lequel le support (93) peut être déplacé le long d'un trajet courbe (96) par le dispositif d'entraînement (106).

7. Dispositif de mesure (80) selon la revendication 6, dans lequel l'analyseur (134) et le dispositif d'entraînement (106) sont reliés à un dispositif de commande (104) et le dispositif de commande (104) peut être mis en fonctionnement pour contrôler l'instant d'acquisition d'un spectre à des fins d'adaptation à la longueur moyenne du trajet de la lumière à travers l'échantillon en fonction de la position du support (93) le long du trajet courbe (96) et/ou dans lequel la position du trajet courbe (96) peut être réglée par un autre dispositif d'entraînement (112) à des fins d'adaptation à la longueur moyenne du trajet de la lumière à travers l'échantillon.

8. Dispositif de mesure (80) selon la revendication 7, dans lequel le support (93) est coudé et comprend une première partie (124) dans laquelle est disposé l'élément et une seconde partie (126) s'étendant transversalement à celle-ci et transversalement à la direction de passage.

9. Moissonneuse-batteuse (10) comportant un dispositif de mesure (80) selon l'une des revendications précédentes.
